(19) Europäisches Patentamt
European Patent Office
Office européen des brevets

(11) EP 2 902 806 B1

(12) EUROPEAN PATENT SPECIFICATION

(45) Date of publication and mention of the grant of the patent:
27.01.2021 Bulletin 2021/04

(51) Int Cl.:
G01T 1/161 (2006.01)          A61B 6/03 (2006.01)
A61B 6/00 (2006.01)          G01T 1/29 (2006.01)
G06T 11/00 (2006.01)

(21) Application number: 13840867.9

(22) Date of filing: 26.09.2013

(86) International application number:
PCT/JP2013/076161

(87) International publication number:
WO 2014/051013 (03.04.2014 Gazette 2014/14)

(54) **NUCLEAR MEDICINE DIAGNOSTIC DEVICE AND MEDICAL DATA PROCESSING DEVICE**

NUKLEARMEDIZINISCHE DIAGNOSTISCHE VORRICHTUNG UND VORRICHTUNG ZUR VERARBEITUNG VON MEDIZINISCHEN DATEN

DISPOSITIF DE DIAGNOSTIC DE MÉDECINE NUCLÉAIRE ET DISPOSITIF DE TRAITEMENT DE DONNÉES MÉDICALES

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 28.09.2012 US 201213630787
20.09.2013 JP 2013196195

(43) Date of publication of application:
05.08.2015 Bulletin 2015/32

(73) Proprietor: Toshiba Medical Systems Corporation
Tochigi 324-8550 (JP)

(72) Inventors:
• WANG, Wenli
Vernon Hills, Illinois 60061 (US)
• GAGNON, Daniel
Vernon Hills, Illinois 60061 (US)
• NIU, Xiaofeng
Vernon Hills, Illinois 60061 (US)

(74) Representative: Moreland, David et al
Marks & Clerk LLP
Aurora
120 Bothwell Street
Glasgow G2 7JS (GB)

(56) References cited:
WO-A1-2011/125181          JP-A- 2012 103 179
JP-A- 2012 145 419          US-A1- 2007 106 154
US-A1- 2010 040 197

• MAURIZIO CONTI: "Tailoring PET Time Coincidence Window Using CT Morphological Information", IEEE TRANSACTIONS ON NUCLEAR SCIENCE, IEEE SERVICE CENTER, NEW YORK, NY, US, vol. 54, no. 5, 2 October 2007 (2007-10-02), pages 1599-1605, XP011194135, ISSN: 0018-9499, DOI: 10.1109/TNS.2007.901230
• JONATHAN K POON ET AL: "Paper;Optimal whole-body PET scanner configurations for different volumes of LSO scintillator: a simulation study;Optimal whole-body PET scanner configurations for different volumes of LSO scintillator: a simulation study", PHYSICS IN MEDICINE AND BIOLOGY, INSTITUTE OF PHYSICS PUBLISHING, BRISTOL GB, vol. 57, no. 13, 7 June 2012 (2012-06-07), pages 4077-4094, XP020225166, ISSN: 0031-9155, DOI: 10.1088/0031-9155/57/13/4077

**Description**

FIELD

Embodiments described herein relate generally to a nuclear medicine diagnosis apparatus and a medical data processing apparatus for imaging an object using radiation detectors, such as gamma cameras or positron emission tomography (PET) scanners.

BACKGROUND

The use of gamma ray detectors in general, that of positron emission tomography (hereinafter referred to as PET) or PET detectors in particular, is growing in the field of medical imaging. In PET imaging, a radiopharmaceutical agent is introduced into an object to be imaged via injection, inhalation, or ingestion. After administration of the radiopharmaceutical, the physical and bio-molecular properties of the agent will cause it to concentrate at specific locations in the human body. The actual spatial distribution of the agent, the intensity of the region of accumulation of the agent, and the kinetics of the process from administration to its eventual elimination are all factors that may have clinical significance. During this process, a positron emitter (radionuclide) attached to the radiopharmaceutical agent will emit positrons according to the physical properties of the isotope, such as half-life, branching ratio, etc.

The radionuclide emits positrons, and when an emitted positron collides with an electron, an annihilation event occurs, wherein the positron and electron are destroyed. Most of the time, an annihilation event produces two gamma rays (at 511 keV) traveling at substantially 180 degrees apart.

By detecting the two gamma rays, and drawing a line between their locations, i.e., the line-of-response (LOR), one can retrieve the likely location of the original disintegration. While this process will only identify a line of possible interaction, by accumulating a large number of those lines, and through a tomographic reconstruction process, the original distribution (radionuclide distribution) can be estimated. In addition to the location of the two scintillation events, if accurate timing (within few hundred picoseconds) is available, a time-of-flight (TOF) calculation can add more information regarding the likely position of the event along the line. Limitations in the timing resolution of the scanner will determine the accuracy of the positioning along this line.

For a clinical, whole-body PET scanner, the imaging field of view (FOV) is a cylindrical volume, with a centered circular region in the transverse plane whose diameter is less than the bore size of the scanner, and the same axial length as the PET scanner. For conventional PET scanners, the traverse FOV, which is the diameter of the circular region in the transverse plane, is typically one of two possible values, e.g., 256 mm for a brain scan, and approximately 576-700 mm for a whole-body scan. Moreover, for each of these two possible PET FOVs, the same fixed coincidence window (in the range of 4-6 ns) is used.

In contrast, as shown in Table 1, conventional X-ray computed tomography (CT) systems support multiple FOVs. 1.

TABLE 1

| CT FOV | S | M | L | LL | XL | XXL |
|---|---|---|---|---|---|---|
| Diameter (mm) | 240 | 320 | 400 | 550 | 700 | 850 |

A problem with conventional PET scanners is that the predetermined whole-body FOV is inadequate for the entire range of patients having different sex, age, body type, and size. Moreover, since an associated CT scan is typically used to obtain the anatomical information of a patient, the different FOV settings used for CT should be adopted for PET. However, using the same fixed coincidence window for different FOVs in PET is inadequate since a large coincidence window for a small FOV increases random coincidences in the prompt data, which degrades the image quality and quantification.

Maurizio Conti: "Tailoring PET Time Coincidence Window Using CT Morphological Information", IEEE Transactions on Nuclear Science, IEEE Service Center, New York, NY, US, vol. 54, no. 5, 1 October 2007 (2007-10-01), pages 1599-1605, discloses a method to reduce the electronic time coincidence window in PET, based on a priori morphological information obtained via a CT scan. A different time coincidence window is assigned to each line of response based on the CT mu-map. A minimum and a maximum time-of-flight are computed based on the position of the patient in the field of view, and data are rejected if their time-of-flight is not compatible with the accepted range.

Jonathan K Poon et al: "Paper; Optimal whole-body PET scanner configurations for different volumes of LSO scintillator: a simulation study Physics in Medicine and Biology, Institute of Physics Publishing, Bristol GB, vol. 57, no. 13, 7 June 2012 (2012-06-07), pages 4077-4094, discloses the use of Monte Carlo simulations to find an optimal scanner geometry (i.e. AFOV, detector thickness and acceptance angle) based on count-rate performance for a range of scintillator volumes ranging from 10 to 90 1 with detector thickness varying from 5 to 20 mm.

US 2007/106154 discloses a method for acquiring PET images with reduced time coincidence window limits that includes obtaining a preliminary image of a patient within a radiation field of view (FOV), determining the spatial location of the patient within the FOV based on the preliminary image, calculating a different time coincidence window based on the spatial location of the patient for each possible pair of oppositely disposed detectors, scanning the patient with a PET scanning system to detect a pair of gamma photons produced by an annihilation event, determining whether the detection of the pair of gamma photons occurs within the time coincidence window, accepting the detected event only if the detection of the gamma photons occurs within the time coincidence window, calculating the spatial location of accepted annihilation event and adding the calculated spatial location of the annihilation event to a stored distribution of calculated annihilation event spatial locations representing the distribution of radioactivity in the patient.

US 2010/040197 discloses an imaging system comprises: a ring of positron emission tomography (PET) detectors, a PET housing at least partially surrounding the ring of PET detectors and defining a patient aperture of at least 80 cm, a coincidence detection processor or circuitry configured to identify substantially simultaneous 511 keV radiation detection events corresponding to electron-positron annihilation events and a PET reconstruction processor configured to reconstruct into a PET image the identified substantially simultaneous 511 keV radiation detection events based on lines of response defined by the substantially simultaneous 511 keV radiation detection events.

According to an aspect of the present invention there is provided a positron emission tomography apparatus as claimed in claim 1.

BRIEF DESCRIPTION OF DRAWINGS

FIG. 1 relates to the present embodiment and illustrates various views of a PET field of view;
FIG. 2 relates to the present embodiment and is a flowchart showing an example of the step of computing a PET coincidence window;
FIG. 3 relates to the present embodiment and illustrates an appearance of a PET-CT scanner system; and
FIG. 4 relates to the present embodiment and illustrates components of the PET-CT scanner system of FIG. 3.

DETAILED DESCRIPTION

Embodiments described herein relate to a new method of determining a coincidence window based on the set FOV for a PET scanner.

In particular, the same FOV settings that are supported in an associated CT scanner are also supported by the corresponding PET scanner in a PET/CT system. Moreover, each PET FOV has a corresponding optimal coincidence window size. The coincidence window (coincidence time width) is determined by the largest time-of-flight difference for the longest oblique line-of-response (LOR) with an emission point located at an edge of the FOV, taking account of the measurement uncertainty in the time-of-flight difference. Thus, the optimal coincidence window size depends on the PET scanner's transverse FOV, axial length, and also on the PET scanner's time-of-flight resolution.

The embodiments are not limited to the PET/CT system. Namely, the embodiments may be applied to a discrete nuclear medicine diagnosis apparatus including a PET/MR or PET device.

Disclosed a method of determining a coincidence window for imaging a region of interest of an object using a Positron Emission Tomography (PET) scanner, the method comprising: (1) determining a diameter of a transverse field of view (FOV) for imaging the region of interest of the object; and (2) calculating the coincidence window based on the determined diameter, a ring diameter of the PET scanner, an axial length of the PET scanner, and a time-of-flight resolution of the PET scanner.

The determining step can include determining the diameter of the transverse FOV from a computed tomography (CT) scanogram of the region of interest of the object.

In one embodiment, the calculating step includes calculating the coincidence window ($\tau$) using the following equation:

$$\tau = \frac{d(mm)}{300(mm/ns)}\sqrt{1+\left(\frac{L}{D}\right)^2} + \frac{n \cdot FWHM_{\Delta TOF}}{2.355}$$

where D is the ring diameter of the PET scanner, L is the axial length of the PET scanner, d is the diameter of the

transverse FOV, FWHM$_{\Delta TOF}$ is the time-of-flight resolution (time resolution of the TOF) of the PET scanner, and n is a predetermined number (2 ≤n ≤ 3).

The axial length L corresponds to the length defined by a plurality of PET detectors along the major axis direction of a top plate in the PET scanner. The ring diameter D corresponds to the diameter of the ring defined by a plurality of PET detectors arranged circularly around the top plate. The diameter d corresponds to the diameter of the FOV in a PET scan. FWHM is a full width at half maximum. FWHM corresponds to, for example, the width of the time-of-flight difference between two points corresponding to a half value of the maximum value (ΔTOF) in the distribution of TOFs generated based on the output of PET detectors to be described later.

A PET scan of the region of interest of the object can be performed using the PET scanner using the calculated coincidence window.

Also disclosed is a method of determining a PET coincidence window for imaging a region of interest of an object using a combined Positron Emission Tomography (PET)-Computed Tomography (CT) scanner, the method comprising: (1) setting a CT field of view (FOV) based on a size of the region of interest of the object; (2) setting a diameter of a transverse FOV for PET imaging to be equal to the set CT field of view; and (3) calculating the PET coincidence window based on the determined diameter, a ring diameter of the PET scanner, an axial length of the PET scanner, and a time-of-resolution of the PET scanner.

According to another embodiment, there is provided a combined Positron Emission Tomography (PET)-Computed Tomography (CT) scanning apparatus, the apparatus comprising: (1) a CT scanner configured to perform a CT scan of a region of interest of an object using a CT field of view (FOV), which is set based on a size of the region of interest of the object; (2) a PET scanner configured to perform a PET scan of the region of interest of the object using a PET FOV having a diameter in a transverse plane, wherein the diameter is determined from the CT scan of the region of interest; and

the PET scanner includes a controller configured to calculate a coincidence window based on the determined diameter, a ring diameter of the PET scanner, an axial length of the PET scanner, and a time-of-flight resolution of the PET scanner.

According to another embodiment, there is provided a combined Positron Emission Tomography (PET)-Computed Tomography (CT) scanning apparatus, the apparatus comprising: (1) a CT scanner configured to perform a CT scan of a region of interest of an object using a CT field of view (FOV), which is set based on a size of the region of interest of the object; (2) a PET scanner configured to perform a PET scan of the region of interest of the object using a PET FOV having a diameter in a transverse plane. The PET scanner sets the diameter of the PET FOV in the transverse plane to be equal to the set CT FOV. The PET scanner includes that a controller calculates a coincidence window based on the set diameter, a ring diameter of the PET scanner, an axial length of the PET scanner, and a time-of-flight resolution of the PET scanner.

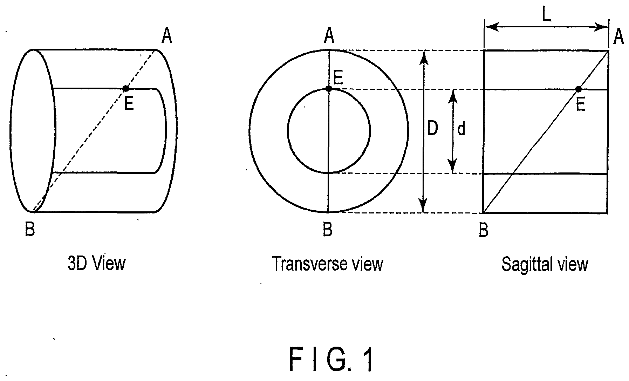

Turning now to the figures, Figure 1 illustrates the imaging FOV for a clinical, whole-body PET scanner. For a cylindrical volume FOV having diameter d within a ring scanner of diameter D and axial length L, the largest time-of-flight difference (ΔTOF) is determined by the longest oblique LOR AB with emission location E at an edge of the FOV, i.e.:

$$\left|\Delta TOF\right|_{max} = \frac{\left|BE - AE\right|}{c} = \frac{d}{c}\sqrt{1+\left(\frac{L}{D}\right)^2}$$

where c is speed of light (300 mm/ns).

The coincidence timing window τ, i.e., the threshold to filter coincidence events by two detected photon's arrival timing difference, must consider both the maximum TOF difference and the timing uncertainty. Under the assumption that the time-of-flight difference ΔTOF is Gaussian distributed, with a standard deviation σ$_{\Delta TOF}$ equal to

$$\sigma_{\Delta TOF} = FWHM_{\Delta TOF} / 2.355$$

then the coincidence window for a cylindrical FOV is computed by:

$$\tau = \left|\Delta TOF\right|_{max} + n\sigma_{\Delta TOF}$$

where n = 2~3 indicates the confidence interval that all true LORs within the cylindrical FOV are detected by the coincidence window. When n = 3, 99.7% of the true LORs within the cylinder are detected by the computed coincidence window. When n = 2, the confidence interval is 95%. A value of n between 2 and 3 is recommended.

Based on the above equations, we have:

$$\tau(ns) = \frac{d(mm)}{300(mm/ns)}\sqrt{1+\left(\frac{L}{D}\right)^2} + \frac{n \cdot FWHM_{\Delta TOF}(ns)}{2.355}$$

Thus, the coincidence window is a function of the FOV (d) (in the PET scanner) and is computed explicitly using the PET detector's diameter (ring diameter: D), length (axial length: L), and timing resolution (FWHM$_{\Delta TOF}$).

The embodiments in which the coincidence window is calculated based on the FOV and the PET detector's properties have advantages over conventional scanners. For example, the amount of random coincidence is reduced for each FOV with the above-calculated "optimal" coincidence window instead of a fixed one, while true coincidences are not affected.

For example, Table 2 lists the computed coincidence window (using D = 909mm, L = 196mm, FWHM ΔTOF = 450ps, and n = 2.7), and the amount of random reduction for different size FOVs using a NEMA-NU2 count-rate phantom, compared with a fixed 3.0 ns coincidence window.

TABLE 2

| PET FOV | S(240) | M(320) | L(400) | LL(550) | XL(700) |
|---|---|---|---|---|---|
| coincidence window (ns) | 1.4 | 1.7 | 2.0 | 2.5 | 3.0 |
| Random reduction | 53% | 43% | 33% | 17% | 0 % |

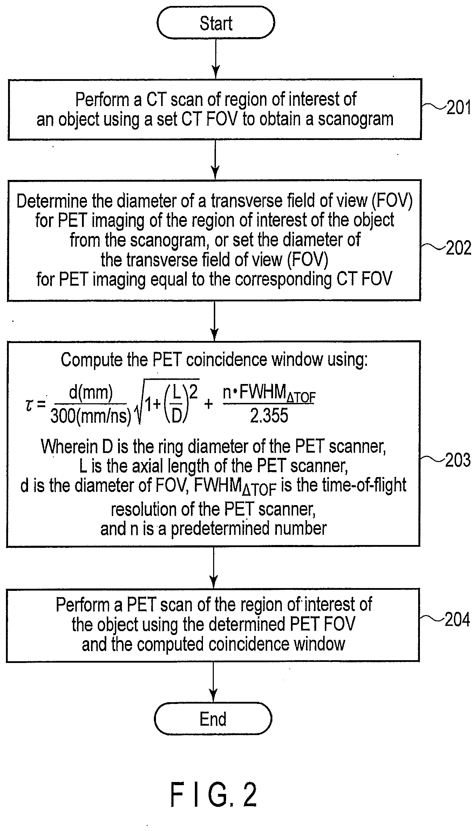

Figure 2 illustrates a method of performing a PET scan with a PET scanner of a PET-CT scanner according to one embodiment.

In step 201, a CT scan of a region of interest of an object is performed using a set CT FOV to obtain a CT scanogram. As shown in Table 1, any of a number of CT FOVs can be used depending on the region of interest and the object being imaged.

In step 202, the diameter d of the transverse FOV for PET imaging is determined. The diameter can be determined from the CT scanogram obtained in step 201. Alternatively, the diameter can be set to be the same as the CT FOV value used in step 201.

In step 203, the PET coincidence window is determined using the formula:

$$\tau = \frac{d(mm)}{300(mm/ns)}\sqrt{1+\left(\frac{L}{D}\right)^2} + \frac{n \cdot FWHM_{\Delta TOF}}{2.355}$$

where D is the ring diameter of the PET scanner, L is the axial length of the PET scanner, d is the diameter of the transverse FOV, FWHM$_{\Delta TOF}$ is the time-of-flight resolution of the PET scanner, and n is a predetermined number.

In step 204, a PET scan of the region of interest of the object is performed using the PET FOV determined in step 202 and the coincidence window computed in step 203.

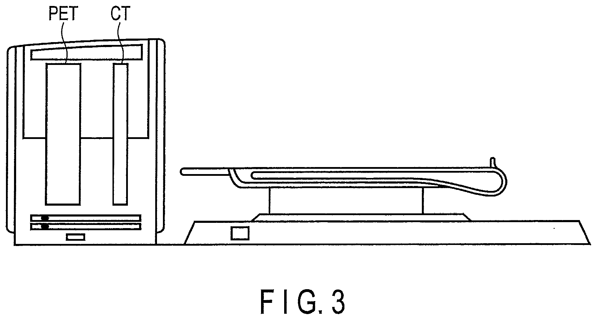

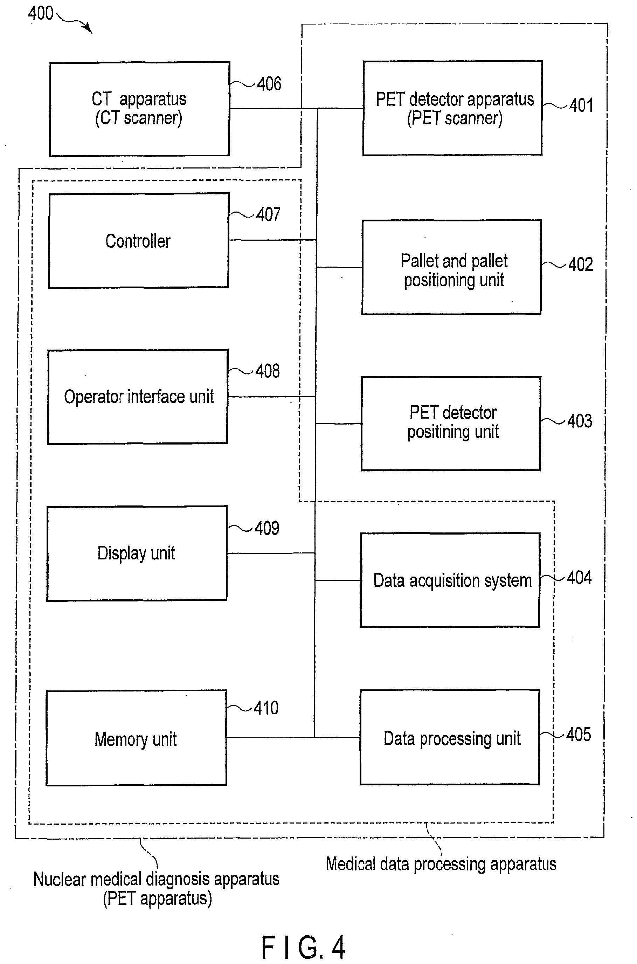

Figures 3 and 4 illustrate a PET/CT system 400 according to one embodiment, the system including a CT apparatus 406 configured to perform a CT scan and a PET detector apparatus (PET scanner) 401 configured to perform a PET scan.

The PET detectors (PET scanners) 401 are arranged around the top plate with a cylindrical shape having a predetermined axial length along a major axis of the top plate and a predetermined ring diameter, and include a plurality of radiation detectors which detect radiation generated in the object.

The PET/CT system 400 includes a movable patient pallet (top plate) 402 that includes a pallet (top plate) positioning unit configured to position the patient pallet (top plate) within the PET detector apparatus 401 and the CT apparatus (CT scanner) 406, based on, for example, commands received from the controller 407.

The controller 407 controls the overall functioning of the PET/CT system 406, including controlling the position of the patient pallet (top plate) via the pallet positioning unit. The pallet positioning unit includes mechanisms configured to move the patient pallet at least in a major axis direction. The controller 407 also controls the PET detector positioning unit 403, which positions one or more PET detector portions around a patient on the pallet.

The controller 407 is also configured to calculate a coincidence window based on the determined diameter, a ring diameter of the PET scanner, an axial length of the PET scanner, and a time-of-flight resolution of the PET scanner, as discussed above.

The controller 407 calculates a coincidence time width for identifying a radiation generation point based on the diameter of the field of view for the object, the axial length, the ring diameter, and the radiation time-of-flight resolution. The controller 407 outputs the calculated coincidence time width to a data processing unit to be described later. The controller 407 controls the PET scanner 401 to perform a PET scan on an object using the calculated coincidence time width.

The controller 407 may determine the diameter of the FOV in the PET scan based on a scanogram generated by performing X-ray computed tomography on the object. The controller 407 may determine the diameter of the FOV in the PET scan to be equal to the FOV in the X-ray computed tomography.

The data acquisition unit 404 obtains PET event data from the PET detector apparatus 401 during a PET scan and sends the event data to the data processing unit for reconstruction of a PET image. The PET event data can also be stored in the memory unit 410 prior to processing by the data processing unit 405.

The operator interface unit 408 is configured to receive operator commands, for example, initiating a CT scan or a PET scan or setting a region of interest on a CT image, and/or to receive parameters associated with the scans. PET and CT images of the patient, as well as operational parameters associated with the scans are displayed on the display unit 409.

As one of ordinary skill in the art would recognize, the controller 407 and the data processing unit 405 can include a CPU that can be implemented as discrete logic gates, as an Application Specific Integrated Circuit (ASIC), a Field Programmable Gate Array (FPGA) or other Complex Programmable Logic Device (CPLD). An FPGA or CPLD implementation may be coded in VHDL, Verilog or any other hardware description language and the code may be stored in an electronic memory directly within the FPGA or CPLD, or as a separate electronic memory. Further, the memory unit 410 may be non-volatile, such as ROM, EPROM, EEPROM or FLASH memory. The memory unit 410 can also be volatile, such as static or dynamic RAM, and a processor, such as a microcontroller or microprocessor, may be provided to manage the electronic memory as well as the interaction between the FPGA or CPLD and the memory unit.

The memory unit 410 stores radiation detection data (PET event data) generated by a scan for detecting radiation in an object mounted on the top plate.

Alternatively, the CPU in the controller 407 or the data processing unit 405 may execute a computer program including a set of computer-readable instructions that perform the functions described herein, the program being stored in any of the above-described non-transitory electronic memories and/or a hard disk drive, CD, DVD, FLASH drive or any other known storage media. Further, the computer-readable instructions may be provided as a utility application, background daemon, or component of an operating system, or combination thereof, executing in conjunction with a processor, such as a Xenon processor from Intel of America or an Opteron processor from AMD of America and an operating system, such as Microsoft VISTA, UNIX, Solaris, LINUX, Apple, MAC-OS and other operating systems known to those skilled in the art.

The data processing unit 405 generates a distribution image of radionuclide injected into the object based on the radiation detection data and the coincidence time width.

Once processed by the data processing unit 405, the processed signals are stored in memory unit 410, and/or displayed on display unit 409. As one of ordinary skill in the art would recognize, memory unit 410 can be a hard disk drive, CD-ROM drive, DVD drive, FLASH drive, RAM, ROM or any other electronic storage known in the art. Display unit 409 can be implemented as an LCD display, CRT display, plasma display, OLED, LED or any other display known in the art. As such, the descriptions of the memory unit 410 and the display unit 409 provided herein are merely exemplary and in no way limit the scope of the present advancements.

When the technical idea of the apparatus of the present embodiment is realized by a nuclear medicine diagnosis apparatus (such as a PET apparatus), the nuclear medicine diagnosis apparatus includes, for example, the structural elements in the alternate long and short dash line in FIG. 4. In this case, each of the steps for determining the coincidence time width (coincidence window) corresponds to each of the steps in the flowchart of FIG. 2, respectively. Those steps are the same as the ones described in the above embodiment.

When the technical idea of the apparatus of the present embodiment is realized by a medical data processing apparatus, the medical data processing apparatus includes, for example, the structural elements in the dashed line. In this case, each of the steps for determining the coincidence time width (coincidence window) corresponds to each of the steps in the flowchart of FIG. 2, respectively. Those steps are the same as the ones described in the above embodiment.

While certain embodiments have been described, these embodiments have been presented by way of example only, and are not intended to limit the scope of the inventions. Indeed the novel methods and systems described herein may be embodied in a variety of other forms; furthermore, various omissions, substitutions, and changes in the form of the methods and systems described herein may be made without departing from the scope of the claims.

## EP 2 902 806 B1

**Claims**

1. A positron emission tomography apparatus, comprising:

   a scanner (401) including a plurality of radiation detectors configured to detect radiation generated in an object and arranged around a top plate with a cylindrical shape having a predetermined axial length along a major axis direction of the top plate and a predetermined ring diameter; and
   a controller (407) configured:

   to determine a largest time-of-flight difference of the radiation by a longest oblique line-of-response with an emission location at an edge of a field of view for the object based on the axial length, the ring diameter, and a diameter of the field of view for the object; and
   to calculate an optimal coincidence time width based on the oblique line of response the largest time-of-flight difference and a standard deviation of a time-of-flight difference of the radiation;
   wherein the controller is configured to determine the diameter of the field of view based on a scanogram generated by performing X-ray computed tomography on the object.

2. The apparatus of claim 1, wherein
   the controller is configured to calculate the optimal coincidence time width ($\tau$) using the following equation:

$$\tau = \frac{d(mm)}{300(mm/ns)} \sqrt{1 + \left(\frac{L}{D}\right)^2} + \frac{n \cdot FWHM_{\Delta TOF}}{2.355}$$

   where D is the ring diameter, L is the axial length, d is the diameter of the field of view, $FWHM_{\Delta TOF}$ is the time-of-flight resolution of the radiation, and n is a predetermined number.

3. The apparatus of claim 2, wherein the predetermined number n is equal to or more than 2 and equal to or less than 3.

4. The apparatus of claim 1, wherein
   the controller is configured to control the scanner to perform a scan on the object using the calculated optimal coincidence time width.

5. The apparatus of claim 1, wherein
   the controller is configured to set the diameter to be equal to a diameter of a field of view in X-ray computed tomography for the object.


**Patentansprüche**

1. Vorrichtung zur Positronen-Emissions-Tomographie, umfassend:

   einen Scanner (401), einschließend eine Mehrzahl von Strahlungsdetektoren, die konfiguriert sind, um in einem Objekt erzeugte Strahlung zu detektieren, und die angeordnet sind um eine obere Platte mit einer zylindrischen Form, die eine vorbestimmte axiale Länge entlang einer Hauptachsenrichtung der oberen Platte und einen vorbestimmten Ringdurchmesser aufweist; und
   eine Steuereinheit (407), die konfiguriert ist:

   um eine größte Time-of-Flight-Differenz der Strahlung mit Hilfe einer längsten schräg einfallenden Line-of-Response mit einer Emissionslage an einem Rand des Sichtbereichs für das Objekt basierend auf der axialen Länge, dem Ringdurchmesser und einem Durchmesser des Sichtbereichs des Objekts zu bestimmen; und
   um eine optimale Koinzidenzzeitbreite basierend auf der größten Time-of-Flight-Differenz und einer Standardabweichung einer Time-of-Flight-Differenz der Strahlung zu berechnen;

   wobei
   die Steuereinheit konfiguriert ist, um den Durchmesser des Sichtbereichs basierend auf ein Scanogramm zu bestimmen, das durch Ausführen von Röntgen-Computertomographie an dem Objekt erzeugt wird.

2. Vorrichtung nach Anspruch 1, wobei die Steuereinheit konfiguriert ist, um die optimale Koinzidenzzeitbreite (τ) unter Verwendung der folgenden Formel zu berechnen:

$$\tau = \frac{d(mm)}{300(mm/ns)}\sqrt{1+\left(\frac{L}{D}\right)^2} + \frac{n \cdot FWHM_{\Delta TOF}}{2.355}$$

worin D der Ringdurchmesser ist, L die axiale Länge ist, d der Durchmesser des Sichtbereichs ist, FWHM$_{\Delta TOF}$ die Time-of-Flight-Auflösung der Strahlung ist und n eine vorbestimmte Zahl ist.

3. Vorrichtung nach Anspruch 2, wobei die vorbestimmte Zahl n gleich oder größer als 2 und gleich oder kleiner als 3 ist.

4. Vorrichtung nach Anspruch 1, wobei die Steuereinheit konfiguriert ist, um den Scanner so zu steuern, dass er einen Scan an dem Objekt ausführt, indem die berechnete optimale Koinzidenzzeitbreite verwendet wird.

5. Vorrichtung nach Anspruch 1, wobei die Steuereinheit konfiguriert ist, um den Durchmesser gleich einem Durchmesser eines Sichtbereichs in einer Röntgen-Computertomographie für das Objekt einzustellen.

**Revendications**

1. Appareil de tomographie par émission de positrons, comprenant :

un scanner (401) incluant une pluralité de détecteurs de rayonnement configurés pour détecter un rayonnement produit dans un objet et agencés autour d'une plaque supérieure dotée d'une forme cylindrique ayant une longueur axiale prédéterminée le long d'un axe principal de direction de la plaque supérieure et un diamètre d'anneau prédéterminé, et
un dispositif de commande (407) conçu pour :

déterminer une différence de temps de vol du rayonnement la plus grande par une ligne de réponse oblique la plus longue ayant un emplacement d'émission à un bord d'un champ de vision pour l'objet sur la base de la longueur axiale, du diamètre d'anneau, et d'un diamètre du champ de vision pour l'objet, et
pour calculer une fenêtre optimale de coïncidence temporelle sur la base de la différence de temps de vol la plus grande et d'un écart-type d'une différence de temps de vol du rayonnement,

dans lequel
le dispositif de commande est configuré pour déterminer le diamètre du champ de vision sur la base d'un scanogramme produit en réalisant une tomodensitométrie à rayons X sur l'objet.

2. Appareil selon la revendication 1, dans lequel
le dispositif de commande est conçu pour calculer la fenêtre de coïncidence temporelle optimale (τ) en utilisant l'équation suivante :

$$\tau = \frac{d(mm)}{300(mm/ns)}\sqrt{1+\left(\frac{L}{D}\right)^2} + \frac{n \cdot FWHM_{\Delta TOF}}{2.355}$$

dans laquelle D est le diamètre d'anneau, L est la longueur axiale, d est le diamètre du champ de vision, FWHM$_{\Delta TOF}$ est la résolution du temps de vol du rayonnement et n est un nombre prédéterminé.

3. Appareil selon la revendication 2, dans lequel le nombre prédéterminé n est égal ou supérieur à 2 et égal ou inférieur à 3.

4. Appareil selon la revendication 1, dans lequel le dispositif de commande est conçu pour commander le scanner afin d'exécuter un examen sur l'objet en utilisant la fenêtre de coïncidence temporelle optimale calculée.

**5.** Appareil selon la revendication 1, dans lequel le dispositif de commande est configuré pour régler le diamètre de façon à ce qu'il soit égal à un diamètre d'un champ de vision en tomodensitométrie à rayons X de l'objet.

3D View     Transverse view     Sagittal view

F I G. 1

```
                    ┌─────────────┐
                    │    Start    │
                    └──────┬──────┘
                           │
                           ▼
   ┌──────────────────────────────────────────────┐
   │    Perform a CT scan of region of interest of │──201
   │ an object using a set CT FOV to obtain a scanogram │
   └──────────────────────┬───────────────────────┘
                          │
                          ▼
   ┌──────────────────────────────────────────────┐
   │ Determine the diameter of a transverse field of view (FOV) │
   │ for PET imaging of the region of interest of the object │
   │           from the scanogram, or set the diameter of       │──202
   │              the transverse field of view (FOV)            │
   │ for PET imaging equal to the corresponding CT FOV          │
   └──────────────────────┬───────────────────────┘
                          │
                          ▼
```

Compute the PET coincidence window using:

$$\tau = \frac{d(mm)}{300(mm/ns)}\sqrt{1+\left(\frac{L}{D}\right)^2} + \frac{n\cdot FWHM_{\Delta TOF}}{2.355}$$

Wherein D is the ring diameter of the PET scanner,
L is the axial length of the PET scanner,
d is the diameter of FOV, $FWHM_{\Delta TOF}$ is the time-of-flight
resolution of the PET scanner,
and n is a predetermined number — 203

```
                          │
                          ▼
   ┌──────────────────────────────────────────────┐
   │    Perform a PET scan of the region of interest of │
   │       the object using the determined PET FOV      │──204
   │         and the computed coincidence window        │
   └──────────────────────┬───────────────────────┘
                          │
                          ▼
                    ┌─────────────┐
                    │     End     │
                    └─────────────┘
```

# F I G. 2

F I G. 3

400

| CT apparatus<br>(CT scanner) ~406 | | PET detector apparatus<br>(PET scanner) ~401 |
| Controller ~407 | | Pallet and pallet<br>positioning unit ~402 |
| Operator interface unit ~408 | | PET detector<br>positining unit ~403 |
| Display unit ~409 | | Data acquisition system ~404 |
| Memory unit ~410 | | Data processing unit ~405 |

Nuclear medical diagnosis apparatus
(PET apparatus)

Medical data processing apparatus

# F I G. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2007106154 A

- US 2010040197 A

### Non-patent literature cited in the description

- Tailoring PET Time Coincidence Window Using CT Morphological Information. **MAURIZIO CONTI.** IEEE Transactions on Nuclear Science. IEEE Service Center, 01 October 2007, vol. 54, 1599-1605

- Paper; Optimal whole-body PET scanner configurations for different volumes of LSO scintillator: a simulation study Physics. **JONATHAN K POON et al.** Medicine and Biology. Institute of Physics Publishing, 07 June 2012, vol. 57, 4077-4094